# EUROPEAN PATENT APPLICATION

(11) **EP 1 652 833 A1**
(43) Date of publication of application: **03.05.2006**
(21) Application number: 04748225.2
(22) Date of filing: 28.07.2004
(51) Int. Cl.: C07C 41/26, C07C 43/263

(54) **PROCESS FOR PRODUCING 4-HYDROXYDIPHENYL ETHER**

(30) Priority: 07.08.2003 JP 2003206450
(71) Applicant: Sumitomo Chemical Company, Limited, Tokyo 104-8260 (JP)
(72) Inventor: KOMOTO, Ichiro, Ibaraki-shi, Osaka 5670841 (JP); NISHIDA, Sumio, Takarazuka-shi, Hyogo 6650882 (JP)
(74) Representative: Vossius & Partner
(86) International application number: PCT/JP2004/011119
(87) International publication number: WO 2005/014514

(57) **Abstract**

A method for producing 4-hydroxydiphenyl ether,
**characterized by** reacting diphenyl ether with an acylating agent in the presence of an acid catalyst to obtain a 4-acyldiphenyl ether represented by the formula (1): 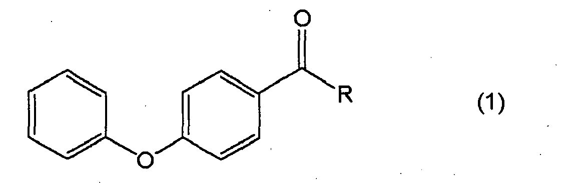 wherein R represents a substituted or unsubstituted alkyl or aryl group,
reacting the 4-acyldiphenyl ether with a peroxide to obtain a 4-acyloxydiphenyl ether represented by the formula (2): 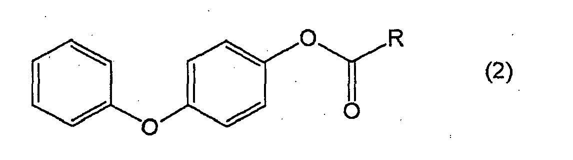 wherein R has the same meaning as defined above, and solvolyzing the ester bond of the 4-acyloxydiphenyl ether, is provided.

## Description

### Technical Field

The present invention provides a method for producing. 4-hydroxydiphenyl ether.

### Background of Invention

4-hydroxydiphenyl ether is a useful compound as the intermediate for pesticides, pharmaceuticals, perfume and plastics. As a method for producing 4-hydroxydiphenyl ether, for example, methods for forming an ether bond among two phenyl groups newly such as a condensation reaction of phenol and a 4-hydroxyhalobenzene (e.g. JP 47-34324 A); a condensation reaction of phenol or a halobenzene and hydroquinone (e.g. JP 55-62033 A and JP 56-135437 A); a condensation reaction of 4-isopropylphenol and halobenzene following a cumene method (e.g. JP 56-59726 A); and a method for carrying out a condensation reaction of phenol and 4-nitrohalobenzene, a reduction of the nitro group, and a diazotization decomposition in sequence (e.g. JP 53-56630 A); are exemplified. However, these methods have problems that high temperature was needed because of low reactivity and they were long process. They have been not satisfactory enough as industrial methods.

### Disclosure of Invention

According to the method of the present invention, it is possible to produce 4-hydroxydiphenyl ether efficiently, using cheep and easily available diphenyl ether as a starting material.

That is, the present invention provides a method for producing 4-hydroxydiphenyl ether, characterized by reacting diphenyl ether with an acylating agent in the presence of an acid catalyst to obtain a 4-acyldiphenyl ether represented by the formula (1): 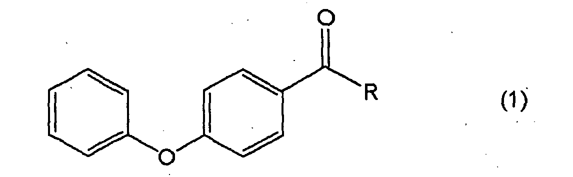 wherein R represents a substituted or unsubstituted alkyl or aryl group,
reacting the 4-acyldiphenyl ether with a peroxide to obtain a 4-acyloxydiphenyl ether represented by the formula (2): 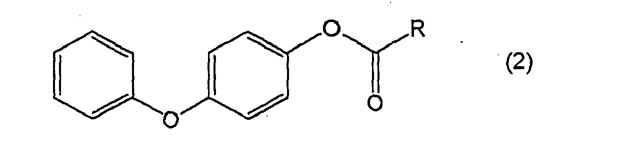 wherein R has the same meaning as defined above, and solvolyzing the ester bond of the 4-acyloxydiphenyl ether.

### Best Mode for Carrying Out the Invention

First, the step for producing a 4-acyldiphenyl ether represented by the formula (1) (hereinafter, simply referred to as the 4-acyldiphenyl ether (1)) by reacting diphenyl ether with an acylating agent in the presence of an acid catalyst will be illustrated.

As the acylating agent, for example, a compound represented by the formula (3):

R-C(O)-X (3)

wherein R represents a substituted or unsubstituted alkyl or aryl group, and X represents a halogen atom (for example, a chlorine or bromine atom) or O-(O)C-R group, herein R represents the same meaning as the above, is exemplified.

Examples of the unsubstituted alkyl groups include C1-10 alkyl groups such as a methyl, ethyl, propyl, butyl, pentyl, hexyl, cyclohexyl, heptyl, octyl, nonyl and decyl group. Examples of the unsubstituted aryl groups include C6-10 phenyl and naphthyl group.

Examples of the substituents of the substituted alkyl groups include aryl groups (for example, a phenyl and naphthyl group), alkoxy groups (for example, a methoxy, ethoxy, propoxy and butoxy group), aryloxy groups (for example, a phenoxy and naphthoxy group), alkylthio groups, arylthio groups, a nitro group, a chlorine atom and a bromine atom.

Examples of the substituents of the substituted aryl groups include alkyl groups (for example, a methyl, ethyl, propyl and butyl group), alkoxy groups (for example, a methoxy, ethoxy, propoxy and butoxy group), aryloxy groups (for example, a phenoxy and naphthoxy group), a nitro group, a chlorine atom and a bromine atom.

More detail, reactive derivatives of a carboxylic acid such as carboxylic halides having an alkyl group having 1 to 10 carbon atoms or an aryl group such as acetyl chloride, acetyl bromide, propionyl chloride, isopropionyl chloride, n-butyryl chloride, isobutyryl chloride, hexanoyl chloride and benzoyl chloride, and carboxylic anhydrides having an alkyl group having 1 to 10 carbon atoms or an aryl group such as acetic anhydride, propionic anhydride, butyric anhydride, isobutyric anhydride and benzoic anhydride, are exemplified.

Among these acylating agents, the carboxylic anhydrides such as acetic anhydride, propionic anhydride, butyric anhydride, isobutyric anhydride and benzoic anhydride, and the carboxylic chlorides such as acetyl chloride, acetyl bromide, propionyl chloride and hexanoyl chloride are preferably used.

In the formula (3), R represents R'-CH₂- group more preferably, herein R' represents a hydrogen atom or a C1-9 alkyl group as the above. That is, the carboxylic anhydrides (for example, acetic anhydride, propionic anhydride and butyric anhydride) wherein the primary carbon of the alkyl group or methyl group is bonded to a carbonyl group and the corresponding carboxylic chloride are even more preferable. Among them, the carboxylic anhydrides (for example, acetic anhydride, propionic anhydride and butyric anhydride) are still more preferable.

As the acid catalyst, a Lewis acid, a Bronsted acid or a mixture thereof is used. Examples of the Lewis acids include metal halides such as aluminum fluoride, aluminum chloride, aluminum bromide, indium chloride, antimony(III) chloride, antimony(V) chloride, zinc chloride, ferrous chloride, ferric chloride, ferric bromide and titanium tetrachloride; metal alkoxides such as titanium tetraisopropoxide, zirconium tetra-tert-butoxide and aluminum triisopropoxide; metal perfluoroalkylsulfonates such as scandium triflate, ytterbium triflate, hafnium triflate, iron(III) triflate, iron(II) triflate, aluminum triflate, gallium triflate, tin(II) triflate, antimony(III) triflate, bismuth(III) triflate, scandium heptadecafluorooctanesulfonate, ytterbium heptadecafluorooctanesulfonate, gallium nonafluorobutanesulfonate, gallium heptadecafluorooctanesulfonate and antimony nonafluorobutanesulfonate; metal bis(perfluoroalkylsulfonyl)amides such as aluminum bis(trifluoromethanesulfonyl)amide and antimony bis(trifluoromethanesulfonyl)amide; metal tris(perfluoroalkylsulfonyl)methides such as scandium tris(perfluoroalkylsulfonyl)methide, aluminum tris(perfluoroalkylsulfonyl)methide and antimony tris(perfluoroalkylsulfonyl)methide; and boron trifluoride.

Examples of the Bronsted acids include perfluoroalkylsulfonic acids such as trifluoromethanesulfonic acid and nonafluorobutanesulfonic acid; bis(perfluoroalkylsulfonyl)imides such as bis(trifluoromethanesulfonyl)imide and bis(nonafluorobutanesulfonyl)imide; tris(perfluoroalkylsulfonyl)methanes such as tris(trifluoromethanesulfonyl)methane and tris(nonafluorobutanesulfonyl)methane; sulfonic acids such as methanesulfonic acid, benzenesulfonic acid and paratoluenesulfonic acid; inorganic acids such as sulfuric acid, hydrogen fluoride, hydrogen chloride, hydrogen bromide, hydrogen iodide and phosphoric acid; metal oxides such as alumina, titania, zirconia and tungsten oxide or a composit thereof; and ion-exchange resins such as Amberlite and Nafion-H (e.g. G. A. Olah ed. "Friedel-Crafts And Related Reactions vol.1-4", Wiley, 1963-1964; and G. A. Olah "Friedel-Crafts Chemistry", Wiley-Interscience, 1973).

The present reaction is usually carried out by mixing diphenyl ether, the acylating agent and the acid catalyst. The mixing order is not particularly limited. The present reaction may be carried out in an atmosphere of an inert gas such as nitrogen gas and argon gas.

The molar ratio of diphenyl ether : the acylating agent is usually 1:5 to 5:1, and the molar ratio can be selected accordingly, and it is preferably the range of about 1:2 to 2:1.

The amount of the acid catalyst to be used is usually 0.0001 to 10 moles, preferably about 0.01 to 5 moles per 1 mole of the acylating agent.

The present reaction may be carried out without using a solvent. The present reaction may be carried out in the presence of an inert solvent. The inert solvent is not limited in so far as it is an inert solvent on the reaction. Examples thereof include halogenated hydrocarbon solvents such as dichloromethane, dichloroethane, chloroform, chlorobenzene, dichlorobenzene and benzotrifluoride; aliphatic hydrocarbon solvents such as pentane, hexane, cyclohexane and heptane; nitrated hydrocarbon solvents such as nitrobenzene, nitromethane and nitroethane; and aprotic solvents such as acetonitrile and carbon disulfide. The solvents may be used alone or in the form of a mixture. The amount of the solvent to be used is not particularly limited.

The reaction temperature is usually the range of about -50 to 150°C, preferably about 0 to 130°C.

After completion of the reaction; for example, an organic layer containing the desirable 4-acyldiphenyl ether (1) can be obtained by adding water or a dilute alkaline water, and if necessary a water-immiscible solvent to the reaction mixture to conduct extracting treatment, and the 4-acyldiphenyl ether (1) can be isolated by concentrating the organic layer. The 4-acyldiphenyl ether (1) isolated may be further purified, if necessary, by distillation, recrystallization or column chromatography.

As R in the 4-acyldiphenyl ether (1) obtained, the same as described in the above-mentioned formula (3) are exemplified.

Specific examples of the 4-acyldiphenyl ether (1) include, for example, 4-acetyldiphenyl ether, 4-propionyldiphenyl ether, 4-butyryldiphenyl ether, 4-isobutyryldiphenyl ether, 4-hexanoyldiphenyl ether, 4-cyclohexanecarbonyldiphenyl ether, 4-benzoyldiphenyl ether, 4-(4'-nitrobenzoyl)diphenyl ether and 4-(4'-chlorobenzoyl)diphenyl ether.

Next, the reaction of the 4-acyldiphenyl ether (1) and the peroxide will be illustrated.

Examples of the peroxides include, for example, organic peracids such as performic acid, peracetic acid, perbenzoic acid metachloroperbenzoic acid, 3,5-dinitroperbenzoic acid, monopermaleic acid, monoperphthalic acid and trifluoroperacetic acid; inorganic peracids such as hydrogen peroxide (for example, aqueous hydrogen peroxide), persulfuric acid, potassium persulfate, percarbonic acid and perphosphoric acid; and a mixture of the peracids such as a mixture of hydrogen peroxide and benzeneperseleninic acid and a mixture of bis(trimethylsilyl)peroxide and trimethylsilyltriflate. Further, it is possible to refer to the description, for example, in Organic Reactions, 9, 73 (1957) and JIKKENN KAGAKU KOUZA, 23, 230 (1991) about the peroxide.

The reaction using the peroxide may be carried out in the air or in an atmosphere of nitrogen gas or argon gas.

The molar ratio of the 4-acyldiphenyl ether (1) : the oxidizing agent is usually in the range of about 1:3 to 3:1, and the reaction can be carried out by selecting preferable amount accordingly.

The reaction using the peroxide is usually carried out using an inert solvent, and the inert solvent is not particularly limited in so far as it is an inert solvent on the reaction. Examples of the inert solvents include halogenated hydrocarbon solvents such as dichloromethane, dichloroethane, chloroform, chlorobenzene, dichlorobenzene and benzotrifluoride; aliphatic hydrocarbon solvents such as pentane, hexane, cyclohexane and heptane; ether solvents such as tetrahydrofuran and tert-butyl methyl ether; nitrated hydrocarbon solvents such as nitrobenzene, nitromethane and nitroethane; aprotic solvents such as acetonitrile and carbon disulfide; carboxylic acids such as acetic acid, propionic acid and trifluoroacetic acid; alcohol solvents such as methanol, ethanol, propanol, isopropanol and tert-butanol; and water. These solvents may be used alone or in the form of a mixture. The amount of the solvent to be used is not particularly limited.

The reaction using the peroxide may be carried out in the presence of an additive such as a base such as sodium hydrogen carbonate, sodium carbonate, potassium hydrogen carbonate, potassium carbonate, sodium acetate, sodium hydroxide and potassium hydroxide, or an acid such as boric acid, sulfuric acid, hydrogen fluoride and paratoluenesulfonic acid. The amount of the additive to be used is not particularly limited, and the amount thereof is usually about 0.01 to 10 moles per 1 mole of the 4-acyldiphenyl ether (1).

The reaction temperature is usually in the range of about -50 to 150°C, preferably about 0 to 100°C.

After completion of the reaction, an organic solvent layer containing the 4-acyloxydiphenyl ether represented by the formula (2) (hereinafter, simply referred to as the 4-acyloxydiphenyl ether (2)) can be usually obtained by adding water, a dilute alkaline water or a dilute acidic water, and if necessary water-insoluble organic solvent to the reaction mixture to conduct extracting treatment.

The organic layer may be used to the solvolysis reaction as it is or the 4-acyloxydiphenyl ether (2) can be isolated by concentrating the organic layer. The 4-acyloxydiphenyl ether (2) isolated may be further purified, if necessary, by distillation, recrystallization or column chromatography.

Specific examples of the 4-acyloxydiphenyl ether (2) include, for example, 4-acetoxydiphenyl ether, 4-propionyloxydiphenyl ether, 4-butyryloxydiphenyl ether, 4-isobutyryloxydiphenyl ether, 4-hexanoyloxydiphenyl ether, 4-cyclohexanecarbonyloxydiphenyl ether, 4-benzoyloxydiplienyl ether, 4-(4'-nitrobenzoyloxy)diphenyl ether and 4-(4'-chlorobenzoylozy)diphenyl ether.

The solvolysis reaction of the ester bond of the 4-acyloxydiphenyl ether (2) is usually carried out in the presence of a protic solvent such as water, an alcohol solvent or a mixture thereof. The reaction is carried out by hydrolyzing the ester bond of the 4-acyloxydiphenyl ether (2) or by transesterification of the ester moiety with the alcohol, i.e. alcoholysis.

Water or the alcohol solvent may be used alone or in the form of a mixture. Examples of the alcohol solvents include, for example, methanol, ethanol, propanol, isopropanol, butanol, sec-butanol and tert-butanol.

The amount of water, the alcohol solvent or the mixture thereof to be used may be equal or more moles per 1 mole of the 4-acyloxydiphenyl ether (2), and there is no specific upper limit.

Water, the alcohol solvent or the mixture thereof may be mixed with the other inert organic solvent to be used. The inert organic solvent is not particularly limited in so far as it is an inert solvent on the reaction. Examples of the inert organic solvents include halogenated hydrocarbon solvents such as dichloromethane, dichloroethane, chloroform, chlorobenzene, dichlorobenzene and benzotrifluoride; aliphatic hydrocarbon solvents such as pentane, hexane, cyclohexane and heptane; ether solvents such as tetrahydrofuran and tert-butyl methyl ether; nitrated hydrocarbon solvents such as nitrobenzene, nitromethane and nitroethane; and aprotic solvents such as acetonitrile, dimethylsulfoxide and dimethylformamide. One kind of the organic solvents or a mixture of two or more kinds of the organic solvents may be mixed with water, the alcohol solvent or the mixture thereof to be used. The amount of the organic solvent is not particularly limited.

The solvolysis reaction is usually carried out under a basic condition or an acidic condition. When the solvolysis reaction is carried out under the basic condition, a Bronsted base or a Lewis base such as sodium hydroxide, potassium hydroxide, sodium carbonate, potassium carbonate, sodium hydrogen carbonate, potassium hydrogen carbonate and ammonia is usually used as the base and the bases are used alone or in the form of a mixture. When the solvolysis reaction is carried out under the acidic condition, a Bronsted acid or a Lewis acid such as sulfuric acid, hydrogen fluoride, hydrogen chloride, hydrogen bromide, acetic acid, phosphoric acid, nitric acid, boric acid, aluminum chloride, aluminum bromide, ferrous chloride, ferric chloride, ferrous bromide, ferric bromide, zinc chloride, zinc bromide, zinc iodide, titanium tetrachloride and boron trifluoride is usually used as the acid and the acids are used alone or in the form of a mixture. The amount of the base or the acid to be used is not particularly limited, and it is usually about 0.001 to 10 moles per 1 mole of the 4-acyloxydiphenyl ether (2).

The reaction temperature of the solvolysis reaction is usually in the range of about 0 to 100°C.

After completion of the solvolysis reaction, an organic layer containing 4-hydroxydiphenyl ether is obtained by adding a water-insoluble organic solvent if necessary to the reaction mixture to conduct extracting treatment. 4-hydroxydiphenyl ether can be isolated by concentrating the organic layer as it is. 4-hydroxydiphenyl ether isolated can be further purified, if necessary, for example, by distillation, recrystallization or column chromatography.

4-hydroxydiphenyl ether can be obtained by solvolyzing the 4-acyloxydiphenyl ether (2) as it is in the reaction system which is produced in the reaction with the peroxide by carrying out the reaction of the 4-acyldiphenyl ether and the peroxide in the presence of the protic solvent used in the solvolysis reaction as the above under the acidic condition by selecting the additive accordingly. In this case, 4-hydroxydiphenyl ether can be isolated by concentrating the organic layer as it is like the above. 4-hydroxydiphenyl ether isolated may be further purified, if necessary, for example, by distillation, recrystallization or column chromatography.

### Examples

The following Examples further illustrate the present invention in detail, but the present invention is not limited by these Examples.

### Example 1

1) Into a 50 mL round-bottomed flask purged with nitrogen, 0.17 g (1.0 mmol) of diphenyl ether, 0.5 mL of orthodichlorobenzene and 0.16 g (1.2 mmol) of aluminum chloride were charged, and 0.09 g (1.1 mmol) of acetyl chloride was added thereto with stirring at 50°C. After the resulting mixture was stirred at the same temperature for 3 hours to effect reaction, the mixture was cooled to room temperature and 10 mL of water was added thereto and extracting treatment was carried out using 20 mL of diethyl ether. After the organic layer was dried over sodium sulfate, sodium sulfate was filtered off and the obtained organic layer was concentrated to obtain an oily matter containing 4-acetyldiphenyl ether. The oily matter was purified by thin layer chromatography (eluent: hexane/ethyl acetate) to obtain 0.21 g of 4-acetyldiphenyl ether. Yield: 98%.
2) Into a 50 mL round-bottomed flask purged with nitrogen, 0.17 g (1.0 mmol) of diphenyl ether, 2 mL of 1,2-dichloroethane and 0.27 g (2 mmol) of aluminum chloride were charged, and 0.12 g (1.2 mmol) of acetic anhydride was added thereto with stirring at 80°C. After the resulting mixture was stirred at the same temperature for 3 hours to effect reaction, the mixture was cooled to room temperature and 10 mL of water was added thereto and extracting treatment was carried out using 20 mL of tert-butyl methyl ether. After the organic layer was dried over sodium sulfate, sodium sulfate was filtered off and the obtained organic layer was concentrated to obtain an oily matter containing 4-acetyldiphenyl ether. The oily matter was determined quantity by high-performance liquid chromatography (hereinafter, simply referred to as LC) to find the yield of 4-acetyldiphenyl ether was 85%.
3) Into a 100 mL round-bottomed flask purged with nitrogen, 8.51 g (50 mmol) of diphenyl ether, 20 mL of 1,2-dichloroethane and 8.0 g (60 mmol) of aluminum chloride were charged, and 4.32 g (55 mmol) of acetyl chloride was added thereto with stirring at 40°C. After the resulting mixture was stirred at the same temperature for 3 hours to effect reaction, the mixture was cooled to room temperature and 100 mL of water was added thereto gradually and extracting treatment was carried out using 100 mL of toluene. After the organic layer was washed twice with 100 mL of water and dried over sodium sulfate, sodium sulfate was filtered off and the obtained organic layer was concentrated to obtain an oily matter containing 4-acetyldiphenyl ether. The oily matter was purified by column chromatography to obtain 9.3 g of 4-acetyldiphenyl ether. Yield: 88%.
4) According to the same manner as that described in the above-mentioned 2), 4-acetyldiphenyl ether was obtained in a yield of 74% (determined quantity by LC) except that 2 mL of chlorobenzene was used in place of 1,2-dichloroethane.
5) According to the same manner as that described in 2), 4-acetyldiphenyl ether was obtained in a yield of 73% (determined quantity by LC) except that 0.19 g (1.2 mmol) of ferric chloride was used in place of aluminum chloride and the reaction temperature was 50°C in place of 80°C.
6) 0.21 g (1.0 mmol) of 4-acetyldiphenyl ether obtained in 3) was dissolved in 2.5 mL of chloroform and 0.26 g (1.5 mmol) of metachloroperbenzoic acid was added thereto and the resulting mixture was stirred at 80°C for 9 hours. After cooling the reaction solution, 10 mL of saturated aqueous sodium hydrogen carbonate solution was added thereto and extracting treatment was carried out using 20 mL of tert-butyl methyl ether and the organic layer obtained was concentrated. The concentrated solution obtained was dissolved in 2.5 mL of methanol and 2 drops of concentrated hydrochloric acid was added thereto and the resulting mixture was stirred at 40°C for 4 hours. This reaction mixture was determined quantity by LC to find the yield of 4-hydroxydiphenyl ether was 75%.

### Example 2

After 4 mL of acetic acid and 0.1 mL of sulfuric acid were added to 0.21 g (1.0 mmol) of 4-acetyldiphenyl ether obtained in 3) of Example 1, 0.25 g (2.2 mmol) of 30% aqueous hydrogen peroxide solution was added thereto and the resulting mixture was stirred at 50°C for 8 hours. The reaction mixture was determined quantity by LC to find the yield of 4-hydroxydiphenyl ether was 73% and 4-acetoxydiphenyl ether was contained therein corresponding to the yield of 11%.

### Industrial Applicability

According to the present invention, 4-hydroxydiphenyl ether, which is useful as the intermediate for pesticides, pharmaceuticals, perfume and plastics, can be produced industrially advantageously from cheep and easily available diphenyl ether.

## Claims

1. A method for producing 4-hydroxydiphenyl ether, **characterized by** reacting diphenyl ether with an acylating agent in the presence of an acid catalyst to obtain a 4-acyldiphenyl ether represented by the formula (1): 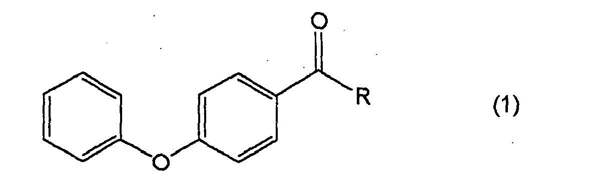 wherein R represents a substituted or unsubstituted alkyl or aryl group,
reacting the 4-acyldiphenyl ether with a peroxide to obtain a 4-acyloxydiphenyl ether represented by the formula (2): 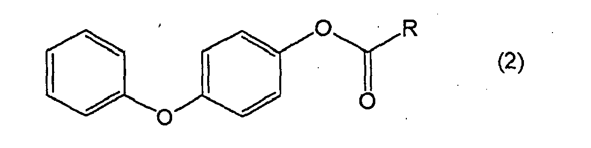 wherein R has the same meaning as defined above, and solvolyzing the ester bond of the 4-acyloxydiphenyl ether.

2. The method for producing according to claim 1, wherein the acylating agent is a carboxylic anhydride.

3. The method for producing according to claim 1 or 2, wherein the acylating agent is the acylating agent wherein the primary carbon of the alkyl group or methyl group is bonded to a carbonyl group.

4. The method for producing according to any one of claims 1 to 3, wherein the amount of the acylating agent to be used is 0.5 to 2 moles per 1 mole of diphenyl ether.

5. The method for producing according to claim 1, wherein the reaction of the 4-acyldiphenyl ether and the peroxide is carried out in the presence of a protic solvent under an acidic condition.

6. The method for producing according to claim 1, wherein the solvolysis of the ester bond of the 4-acyloxydiphenyl ether is carried out in the presence of a protic solvent under an acidic condition.

7. The method for producing according to claim 5, which comprises solvolyzing the 4-acyloxydiphenyl ether (2) produced in the reaction with the peroxide as it is in the reaction system to obtain 4-hydroxydiphenyl ether.

8. The method for producing according to claim 5, wherein the peroxide is an inorganic peroxide.

9. The method for producing according to claim 5 or 7, wherein the peroxide is aqueous hydrogen peroxide.
